# EUROPEAN PATENT SPECIFICATION

(11) **EP 3 151 844 B1**
(45) Date of publication and mention of the grant of the patent: **05.08.2020**
(21) Application number: 15725671.0
(22) Date of filing: 04.06.2015
(51) Int. Cl.: A61K 35/20, A61K 36/899, A61K 35/36, A61K 35/57, A61K 36/185, A61K 36/28, A61K 36/49, A61K 39/00, A61P 37/08

(54) **ALLERGEN FOR PROPHYLACTIC TREATMENT OF ALLERGY**
ALLERGEN ZUR VORBEUGENDEN BEHANDLUNG VON ALLERGIEN
ALLERGÈNE POUR LE TRAITEMENT PROPHYLACTIQUE D'UNE ALLERGIE

(30) Priority: 04.06.2014 EP 14171132
(43) Date of publication of application: 12.04.2017
(73) Proprietor: ALK-Abelló A/S, 2970 Hørsholm (DK)
(72) Inventor: ARSHAD, Syed Hasan, Chilworth Southampton SO16 7JB (GB); ROBERTS, Graham Colin, Winchester SO22 5DT (GB); RIIS, Bente, DK-3450 Allerød (DK); SCHRØDER, Dorte Ellen, DK-1437 Copenhagen K (DK)
(74) Representative: Inspicos P/S
(86) International application number: PCT/EP2015/062496
(87) International publication number: WO 2015/185684

(56) References cited:
- EP-A1- 1 440 979
- EP-A1- 2 626 082
- WO-A1-2008/092902

## Description

### TECHNICAL FIELD OF THE INVENTION

The present invention relates to a composition comprising at least one first allergen belonging to a first biological source material group of the genus Dermatophagoides for prophylactic treatment of allergy.

### BACKGROUND OF THE INVENTION

WO 2010/128364 discloses a method of treatment of pollen allergy, wherein an extract of mountain cedar pollen (Juniperus ashei of the Cupressaceae family) is used to treat patients allergic to Japanese cedar (Cryptomeria japonica).

WO 95/17208 discloses a method of preventing allergic disease to an allergen comprising administering the allergen to an unsensitised individual in order to induce establishment of a stable population of allergen-specific T-helper-1-like memory lymphocytes, which are capable of inhibiting activity or amplification of allergen-specific T-helper-2-like memory lymphocytes stimulating production of IgE specific to said allergen.

Sensitisation is defined to mean that allergen-specific TH-2 cells have not been primed. The individuals treated are indicated to be unsensitised children of between 3 months and 7 years of age, preferably more than 6 months, more preferably more than 9 months old. No further indication of the selection of children to be treated is given. The allergen may be administered by the oral, intranasal, oronasal, rectal, intradermal or subcutaneous route. The allergen may be a single allergen or a combination of allergens, e.g. HDM, grass and cat allergens.

WO 2006/072251 discloses a method of prophylactic treatment of allergy to an allergen comprising administering an allergy composition comprising the allergen as active substance to a subject by oromucosal route, wherein the subject is unsensitised to the allergen of the composition in the sense of exhibiting no IgE response. The subject is preferably between 6 months and 10 years. The subject may be unsensistised to any allergen. Nothing further is indicated about how the subjects for treatment are selected. Preferably, the oromucosal administration is sublingual administration, e.g. daily administration. The formulation may be any formulation suitable for oromucosal administration. Any allergen may be used, e.g. HDM, cat and grass allergen. The allergens may be given singly or in combinations of two or more allergens from different biological sources.

WO 2012/049310 discloses an antigen for treatment and prophylactic treatment of a hypersensitivity immune response in an individual in need thereof, wherein the immune response is triggered by an allergen upon the individual's exposure to a source material comprising said allergen and wherein i) the antigen is unrelated to the allergen(s) triggering the hypersensitivity immune response in the individual and is obtainable from or is derivable from said source material; and ii) the antigen is administered in a therapeutically effective amount to said individual. Prophylactic treatment includes treatment of individuals, before the individual becomes sensitised to an allergen, i.e. before the individual has raised detectable IgE antibodies specific to the antigen, as well as treatment of individuals after sensitisation and before development of clinical symptoms the disesase, such as symptoms of allergic rhinitis, allergic asthma or atopic dermatitis.

WO 2012/049312 discloses an antigen for use in treatment or prophylactic treatment of a hypersensitivity immune response of the respiratory tract in an individual in need thereof, wherein the immune response is triggered by an allergen upon the individual's exposure to an environmental source material comprising said allergen and wherein iv) the antigen is unrelated to the allergen(s) triggering the hypersensitivity immune response in the individual; and v) the antigen is administered in a therapeutically effective amount to the oral cavity of said individual, and vi) the antigen also is administered to the respiratory tract within a period at least coinciding partly or entirely with the individual's exposure to said source material.

WO 2013/156467 discloses a method for treatment or prevention of a hypersensitivity immune response in an individual to a non-profilin allergen of a profilin-containing plant material, comprising administration of a therapeutically effective amount of a profilin polypeptide. Prophylactic treatment includes treatment of individuals, before the individual becomes sensitised to an allergen, i.e. before the individual has raised detectable IgE antibodies specific to the antigen, as well as treatment of individuals after sensitisation and before development of clinical symptoms the disesase, such as symptoms of allergic rhinitis, allergic asthma or atopic dermatitis.

WO 2008/092902 discloses use of a Der f 2 allergen composition for the manaufacture of a composition for preventing or treating allergy to Der p 2 and vice versa. Preventing means any type of prophylactic treatment, including partly or wholloy preventing or inhibiting the development of symptoms or the development of causes of symptoms.

Holt et al., J Allergy Clin Immunol, Vol. 132, Number 4, pages 991-993, discloses a clinical pilot study of prophylactic use of sublingual allergen immunotherapy in high-risk children. Inclusion criteria were age 18-30 months, positive atopic family history coupled with personal history of atopic dermatitis and sensitisation to one or more food allergens, and no sensitisation to treatment allergens. The children were given a mixture of extracts of house dust mite (mixture of Der p 1 and Der f 1), cat (Fel d) and grass pollen (Phi p) in the form of a liquid solution by sublingual route using a modified palate cleft spoon daily for 12 months. Sensitisation levels were measured at 12, 24 an 48 months, and no differences between active and placebo groups were measured.

WO 2006/050729 discloses a method of preventive treatment of allergy to an allergen in a subject comprising administering an allergy composition containing the allergen as active substance to a mucosal surface of the subject, a) wherein the subject to be treated is sensitised so as to exhibit an IgE response specific to the allergen, b) wherein the subject to be treated is free of clinical symptoms of the allergy associated with the allergen, and c) wherein the preventive treatment is aimed at preventing or reducing subsequent clinical symptoms of the allergy associated with the allergen.

Jacobsen et al, Allergy 62, 2007, 943-948, discloses a study of the preventive effect of subcutaneous specific immunotherapy of subjects with grass pollen and/or birch pollen allergy on the development of asthma, and it was found that a 3 year treatment had a long term effect at least 7 years on the development of asthma.

EP-A1-2 626 082 discloses a method of preventing allergies in a mammal comprising 1) detecting in a child the occurrence of a first allergic reaction to a first allergen, 2) administering epicutaneously to the child the said first allergen to protect the child from development of at least one further allergy to another allergen. In particular, EP-A1-2 262 082 discloses in vivo mouse experiments showing that epicutaneous administration of milk proteins to mice allergic to milk desensitises against milk and also against peanut.

EP 1 440 979 A1 discloses preparation of hypoallergenic mosaic proteins for use in allergy vaccines.

### BRIEF DESCRIPTION OF THE INVENTION

The object of the invention is to provide a composition, such as an allergy vaccine, for use in prophylactic treatment of allergy.

This object is obtained with the present invention, which relates to a composition comprising at least one first allergen , for use in prophylactic treatment of allergy to at least one second allergen in a human subject,
wherein the first allergen is a respiratory allergen of a first biological source material group of the genus Dermatophagoides,
wherein the second allergen is selected from the group consisting of a respiratory allergen of a second biological source material group, a venomous allergen of a second biological source material group, and a food allergen of a food composition,
wherein the human subject
   i. has a negative skin prick test to the first allergen and to the second allergen and
   ii. has not developed any clinical symptoms of allergy to the first allergen and to the second allergen, and
wherein the first and second biological source material groups are not from the same order of the taxonomic order system.

The present invention is based on the surprising clinical finding that prophylactic treatment using a composition comprising a house dust mite composition reduces the incidence of sensitisation and clinical symptoms in a test population both for allergy to house dust mite and for allergy to five other allergens, specifically grass pollen, cat, milk, egg and peanut to which the subject was unsensitisedat the start of treatment. Thus, the present clinical study has shown that an allergen composition comprising a house dust mite composition has a prophylactic effect in the sense of preventing or delaying sensitisation and hence onset of clinical symptoms of allergy. Moreover, the present clinical study has surprisingly shown that an allergen composition comprising a house dust mite composition has a prophylactic effect not only for allergy to house dust mite allergens but for inhalation and food allergies in general.

The present invention further enables a method of prophylactic treatment of allergy to at least one second allergen, which is selected from the group consisting of a respiratory allergen belonging to a second biological source material group, a venomous allergen belonging to a second biological source material group, and a food allergen of a food composition, in a subject comprising administering to the subject a composition comprising at least one first allergen defined above, wherein the subject has not developed any clinical symptoms of allergy to the first allergen and to the second allergen, and wherein the first and second biological source material groups are not the same.

### DETAILED DESCRIPTION OF THE INVENTION

### Allergens

The expression "first biological source material group" may designate a genus of the taxonomic order system. Alternatively, the expression may designate a family or an order of the taxonomic order system

The expression "second biological source material group" may designate a group consisting of a genus of the taxonomic order system and a food composition. Alternatively, the expression may designate a group consisting of a family of the taxonomic order system and a food composition. Alternatively, the expression may designate a group consisting of an order of the taxonomic order system and a food composition.

For the respiratory allergen, the expression "first biological source material group" denotes the genus Dermatophagoides from the house dust mite family Pyroglyphidae of the order Astigmata.

### Genera

For the respiratory allergen, the expression "second biological source material group" include genera in the taxonomic order system including
genera of grass family Poaceae (order Poales), e.g. the genera Phleum, Lolium, Poa, Cynodon, Dactylis, Holcus, Phalaris and Secale; genera of nettle family Urticaceae (order Rosales), e.g. genus Parietaria; genera of daisy family Asteraceae (order Asterales), e.g. the genera Ambrosia and Artemisia; genera of birch family Betulaceae (order Fagales), e.g. the genera Betula, Alnus, Corylius and Carpinus; genera of the olive family Oleaceae (order Lamiales), e.g. the genus Olea; genera of the beech family Fagaceae (order Fagales), e.g. genus Quercus; genera of the cypress family Cupressaceae (order Cupressales), e.g. the genera Juniperus and Cryptomeria; genera of the plane-tree family Plantanaceae (order Plantales), e.g. the genus Platanus;
genera of the cockroach family Ectobiidae (order Blattodea), e.g. the genera Blatella and Periplaneta; genera of the midge family Chironomidea (order Diptera), e.g. Chironomus; genera of the flea amily Pulicidae (order Siphonaptera), e.g. Ctenoecephalides; genera of the cat family Felidae (order Carnivora), e.g. the genus Felis; genera of the dog family Canidae (order Carnivora), e.g. the genus Canis;
genera of the fungi/mould family Pleosporaceae (order Pleosporales), e.g. the genus Alternaria; genera of the fungi/mould family Cladosporiaceae (order Capnodiales), e.g. the genus Cladosporium; genera of the horse family Equidae (order Perissodactyla), e.g. the genus Equus; genera of the ox family Bovidae (order Cetartiodactyla), e.g. the genus Bos.

For the venomous allergen, the expression "second biological source material group" include genera in the taxonomic order system including
genera of the bee family Apidae (order Hymenoptera), e.g. the genera Apis; genera of the wasp family Vespidae (order Hymenoptera), e.g. the genera Vespula; genera of the ant family Formicidae (order Hymenoptera), e.g. the genus Solenopsis;

### Families

In a specific embodiment of the invention, the expression "second biological source material group" for the respiratory allergen include families in the taxonomic order system including the grass family Poaceae (order Poales), e.g. of the genera Phleum, Lolium, Poa, Cynodon, Dactylis, Holcus, Phalaris and Secale; the nettle family Urticaceae (order Rosales), e.g. genus Parietaria; the daisy family Asteraceae (order Asterales), e.g. the genera Ambrosia and Artemisia; the birch family Betulaceae (order Fagales), e.g. the genera Betula, Alnus, Corylius and Carpinus; the olive family Oleaceae (order Lamiales), e.g. the genus Olea; the beech family Fagaceae (order Fagales), e.g. genus Quercus; the cypress family Cupressaceae (order Cupressales), e.g. the genera Juniperus and Cryptomeria; the plane-tree family Plantanaceae (order Plantales), e.g. the genus Platanus;
the cockroach family Ectobiidae (order Blattodea), e.g. the genera Blatella and Periplaneta; the midge family Chironomidea (order Diptera), e.g. Chironomus; the flea amily Pulicidae (order Siphonaptera), e.g. Ctenoecephalides; the cat family Felidae (order Carnivora), e.g. the genus Felis; the dog family Canidae (order Carnivora), e.g. the genus Canis;
the fungi/mould family Pleosporaceae (order Pleosporales), e.g. the genus Alternaria; the fungi/mould family Cladosporiaceae (order Capnodiales), e.g. the genus Cladosporium; the horse family Equidae (order Perissodactyla), e.g. the genus Equus; the ox family Bovidae (order Cetartiodactyla), e.g. the genus Bos.

In a specific embodiment of the invention the expression "second biological source material group" for the venomous allergen include families in the taxonomic order system including the bee family Apidae (order Hymenoptera), e.g. the genera Apis; the wasp family Vespidae (order Hymenoptera), e.g. the genera Vespula; the family ant Formicidae (order Hymenoptera), e.g. the genus Solenopsis;

### Orders

In another specific embodiment of the invention, the expression "second biological source material group" for the respiratory allergen includes orders in the taxonomic order system including the order Poales, including the grass family Poaceae, e.g. the genera Phleum, Lolium, Poa, Cynodon, Dactylis, Holcus, Phalaris and Secale; the order Rosales, including the nettle family Urticaceae, e.g. genus Parietaria; the order Asterales, including the daisy family Asteraceae, e.g. the genera Ambrosia and Artemisia; the order Fagales, including the birch family Betulaceae, e.g. the genera Betula, Alnus, Corylius and Carpinus, and the beech family Fagaceae, e.g. genus Quercus; the order Lamiales, including the olive family Oleaceae, e.g. the genus Olea; the order Cupressales, including the cypress family Cupressaceae, e.g. the genera Juniperus and Cryptomeria; the order Plantales, including the plane-tree family Plantanaceae, e.g. the genus Platanus;
the order Blattodea, including the cockroach family Ectobiidae, e.g. the genera Blatella and Periplaneta; the order Diptera, including the midge family Chironomidea, e.g. Chironomus; the order Siphonaptera, including the flea amily Pulicidae, e.g. Ctenoecephalides; the order Carnivora, including the cat family Felidae, e.g. the genus Felis, and the dog family Canidae, e.g. the genus Canis;
the order Pleosporales, including the fungi/mould family Pleosporaceae, e.g. the genus Alternaria; the order Capnodiales, including the fungi/mould family Cladosporiaceae, e.g. the genus Cladosporium; the order Perissodactyla, including the horse family Equidae, e.g. the genus Equus; the order Cetartiodactyla, including the ox family Bovidae, e.g. the genus Bos.

In another specific embodiment of the invention the expression "second biological source material group" for the venomous allergen include orders in the taxonomic order system including
the order Hymenoptera, including the bee family Apidae, e.g. the genera Apis, the wasp family Vespidae, e.g. the genera Vespula, and the family ant Formicidae, e.g. the genus Solenopsis.

### First allergen

The first biological source material originates from the genus Dermatophagoides.

In a particular embodiment of the invention the first allergen comprises or is an allergen selected from the group consisting of Derf 1, Der f 2, Der p 1, Der p 2, Der p 7, and Der m 1.

### Second allergen

The second biological source material group may include both a genus in the taxonomic order system component and a food composition component.

In a specific embodiment, the genera in the taxonomic order system component of the second biological source material group is selected from the group consisting of the genera from the grass family Poaceae, the daisy family Asteraceae, the birch family Betulaceae, the beech family Fagaceae, the cypress family Cupressaceae, the cat family Felidae, the bee family Apidae and the wasp family Vespidae. In a specific embodiment, the genera in the taxonomic order system component of the second biological source material group is selected from the group consisting of the genera from the grass family Poaceae, and the cat family Felidae. In particular, the genera in the taxonomic order system of the second biological source material group is selected from the group consisting of the genera Phleum, Lolium, Poa, Cynodon, Dactylis, Holcus, Sorghum, Phalaris, Secale, Ambrosia, Betula, Alnus, Corylius, Carpinus, Quercus, Juniperus, Cryptomeria, Felis, Apis and Vespula. More particularly, the genera in the taxonomic order system of the second biological source material group is selected from the group consisting of the genera Phleum, Lolium, Poa, Cynodon, Dactylis, Holcus, Sorghum, Phalaris, Secale, and Felis.

In a particular embodiment of the invention the second allergen comprises or is an allergen selected from the group consisting of Bet v 1, Aln g 1, Cor a 1, Car b 1, Que a 1, Cry j 1, Cry j 2, Cup a 1, Cup s 1, Jun a 1, Jun a 2, Jun a 3, Ole e 1, Lig v 1, Pla l 1, Pla a 2, Amb a 1, Amb a 2, Amb t 5, Art v 1, Art v 2 Par j 1, Par j 2, Par j 3, Sal k 1, Cyn d 1, Cyn d 7, Dac g 1, Hol l 1, Lol p 1 and 5, Pha a 1, Pas n 1, Phi p 1, Phi p 5, Phi p 6, Poa p 1, Poa p 5, Sec c 1, Sec c 5, Sor h 1, Eur m 2, Lep d 2, Blo t 1, Tyr p 2, Fel d 1, Can f 1, Can f 2, Alt a 1, Cla h 1 and Asp f 1. In a particular embodiment of the invention the second allergen is selected from the group consisting of Cyn d 1, Cyn d 7, Dac g 1, Hol l 1, Lol p 1 and 5, Pha a 1, Pas n 1, Phi p 1, Phi p 5, Phi p 6, Poa p 1, Poa p 5, Sec c 1, Sec c 5, Sor h 1, Amb a 1, Amb a 2, and Amb t 5.

In a specific embodiment, the food composition part of the second biological source material group is selected from the group consisting of mammalian animal milk, including cows milk, mares' milk, goats' milk and ewes milk; bird eggs, including hens' eggs, e.g. hens' egg white; fish, including cod, mackerel, herring,carp, salmon, tuna and plaice; shellfish (Crustaceans and Mollusca), including shrimp, crab, sqid, prawn, snail, oyster and abelone; peas/beans, including peanuts, soy beans, green beans and green peas; grains, including wheat and rye; additives, including both synthetic and naturally occurring; foods for which birch pollen allergics have cross reactions, including hazelnuts, green apples, peaches, almonds, kiwis, tomatoes and potatoes; foods for which grass pollen allergics have cross reactions, including wheat, rye and corn. In particular, the food composition part of the second biological source material group is selected from the group consisting of hens' egg, cows' milk and peanut.

The allergenic component of the animal food compositions is as follows: shell fish: tropomyosins; fish: paralbumins; mammalian animal milk: casein.

In a particular embodiment of the invention the second allergen is selected from the group consisting of Pen a 1, Met e 1, Pen m 1, Pen i 1, Hel as 1, Tod p 1, Cra g 1, Cra g 2, Cra g 1.03, Cha f 1, Hal d 1, Gad c 1, Cyp c 1.01, Cyp c 1.02, Sal s 1.01, Sal s 1.02, Thu o 1.01, Thu o 1.02, Ran e 1, Ran e 2, Bos d 8 and alfa_{s1*n*}, alfaₛ₂, beta-casein and kappa-casein from cow, goat and sheep.

The allergenic component of plant food compositions may be listed as follows: Prolamins found in cereal seed; non-specific lipid-transfer proteins found in apple, apricot, cherry, peach, plum, strawberry, orange, grape, chestnut, walnut, hazelnut, asparagus, lettuce, cabbage and maize; alfa-amylase/trypsin inhibitors found in barley and rice; albumins found in walnut, almond, brazil nut, cashew nut, white mustard, black mustard, chickpea, peanut, sesame and sunflower; Bet v 1 superfamily substances found in apple, pear, cherry, strawberry, carrot, celery root, hazelnut, soy; 7S (vicilin-like) globulins found in peanut, soy, buckwheat, almond, walnut, hazelnut, cashew nut and sesame; legumin-like globulins found in peanut, soy, buckwheat, almond, cashew nut and brazil nut; and cysteine protease C1 superfamily substances found in kiwi and soy.

In a particular embodiment of the invention the second allergen is selected from the group consisting of alfa- and beta-gliadins, LMW glutenin, Mad d 3, Pru ar 3, Pru av 3, Pru p 3, Pru d 3, Fra a 3, Cit s 3, Vit v 1, Cas s 8, Jug r 3, Cor a 8, Aspa o 1, Lac s 1, Bra o 3, Zea m 14, Hor v 1, Rag 1, 2, 5, 5b, 14, 14b, 16 and 17, Jug r 1, Ber e 1, Ana o 3, Sin a 1, Bra j 1, Ara h 2, Ara h 6, Ara h 7, Ses i 1, Ses i 2, SFA-8; Mal d 1, Pyr c 1, Pru av 1. Dau c 1, Api g 1, Cor a 1.04, Gly m 4, Conarachin, Ara h 1, beta-conglycinin, BWI-1c, BWI-2, BWI-2b, BWI-2c, Conglutin Gamma, Jug r 2, Cor a 11, Ana o 1.0101, Ana o 1.0102, Ses i 3, Ara h 3, Ara h 4, Glycinin, BW24KD, Fag e 1, FAGAG1, FA02, FA18, Major almond protein, amandin, pruning, Ana o 2, Ber e 2, Excelsin, Actinidin, Gly m Bd 30K, P34 and Gly m 1.

In a particular embodiment of the invention, the second biological source material is selected from the group consisting of the genera of the taxonomic system Phleum, Ambrosia and Dermatophagoides, and the food compositions hens' egg, cows' milk and peanut.

### Form of allergen

The present invention has surprisingly shown that treatment with a first allergen from a first biological source material has a prophylactic effect for allergy to a second allergen from a second biological source material, even when the first and second allergens are remote in the taxonomic order system, such as when the first allergen is house dust mite allergens and the second allergen is grass or cat allergen. This is surprising, since the allergens are unrelated in function, amino acid sequence and structure and hence the prophylactic effect is unlikely to be caused by an immunogenic cross-reactivity of the allergens.

The first allergen of the composition for the use of the invention may be an extract of the first biological source material or a single allergen or a mixture of single allergens.

An extract of a biological source material is a complex mixture of a number of allergens, non-allergen proteins, lipids carbohydrates, salts etc. The extract is preferably selected from the group consisting of an allergen extract from the first biological source material, a purified fraction of an allergen extract from the first biological source material, a modified allergen extract, including allergoids and combinations thereof. An allergenic extract may naturally contain a number of different allergens and one or more isoforms of the same allergen.

A single allergen may be an allergen purified from an allergen extract or a recombinantly prepared allergen. The single allergen is preferably selected from the group consisting of an allergen purified from an allergen extract, a native recombinant allergen and a mutated recombinant allergen. A recombinant allergen typically only represents one isoform of an allergen. The mutanted recombinant allergen may be a low IgE-binding mutant, e.g. a low IgE-binding allergen according to WO 99/47680, WO 02/40676 or WO 03/096869.

In a preferred embodiment of the invention, the first allergen is selected from the group consisting of an allergen extract from the first biological source material, a purified fraction of an allergen extract from the first biological source material, a native recombinant allergen and a mutated recombinant allergen.

Allergens may be present in equi-molar amounts or the ratio of one allergen to another in the first allergen may be in the range of 1:20 to 20:1.

### Subject to be treated

In a specific embodiment of the composition for the use of the invention, the subject has a negative skin prick test to the first allergen.

In a specific embodiment of the composition for the use of the invention, the subject has a negative skin prick test to the second allergen.

In a specific embodiment of the composition for the use of the invention, the subject has a negative skin prick test to the first and to the second allergen.

In a specific embodiment of the composition for the use of the invention, the subject does not exhibit a positive IgE response to the first allergen.

In a specific embodiment of the composition for the use of the invention, the subject does not exhibit a positive IgE response to the second allergen.

In a specific embodiment of the composition for the use of the invention, the subject does not exhibit a positive IgE response to the first and to the second allergen.

In a specific embodiment of the composition for the use of the invention, the subject has not yet exhibited any clinical symptoms of allergy or has a negative skin prick test or does not exhibit a positive IgE response to any allergen. Alternatively, the subject exhibits clinical symptoms of allergy or has a positive skin prick test or exhibits a positive IgE response to one or more allergens other than first and second allergens.

In a specific embodiment of the composition for the use of the invention, the subject has not developed any clinical symptoms of allergy or has a negative skin prick test or does not exhibit a positive IgE response to any of the second allergens hens' eggs, cow's milk, peanut and soy bean.

In a specific embodiment of the composition for the use of the invention, the subject has not developed any clinical symptoms of allergy or has a negative skin prick test or does not exhibit a positive IgE response to the allergens house dust mite, grass pollen, cat, hens' eggs, cow's milk and peanut.

In a particular embodiment, the subject treated is selected from the group consisting of a human subject, a cat and a dog. In a particular embodiment, the subject treated is a human subject.

### Clinical symptoms

The subject to be treated has not yet developed any clinical symptoms of the allergy to the first and the second allergen.

The clinical symptoms of the allergy to the allergen are any conventional symptom, including rhinitis, conjunctivitis, rhinorrhea, nasal obstruction, sinusitis, sneezing, atopic dermatitis, itching, watery eyes, watery nose, wheezing and skin irritation. Typical clinical symptoms of food allergy are oral itchting or swelling, nausea, vomiting, abdominal pain, diarrhoea, rhinitis, angio-oedema and urticaria, and life-threatening reactions may include exacerbation of asthma, laryngeal oedema and anaphylaxis. The clinical symptoms of the asthma are reversible airway narrowing, airway hyper-responsiveness and airway inflammation.

A number of factors are indicative for development of allergy with manifested clinical symptoms later in life. In the following subjects exhibiting one or more such indicating factors are referred to as high risk subjects. Indicating factors of high risk subjects are clinical symptoms of allergies associated with one or more allergens other than the first allergen of the composition. Further indicating factors of high risk subjects are a positive skin prick test and/or a positive IgE response to one or more allergens other than the first allergen of the composition. Further indicating factors of high risk subjects are the presence of one or more allergies in one or both parents or grandparents or in one or more sibling. The prophylactic treatment disclosed herein is particularly suitable for high risk subjects. However, the subject to be treated may also be a subject exhibiting no indicating factors of high risk subjects, e.g. free of clinical symptoms of allergy to other allergens.

### Administration routes

The composition for the use of the present invention may be administered by any conventional route used for allergy compositions, including mucosal administration and administration by injection. In a particular embodiment of the invention, the composition is administered by mucosal administration and administration by injection.

The composition for the use of the invention is for use in prophylactic treatment, which is commonly referred to as immunotherapy, and such a composition may be referred to as an allergy vaccine.

### Administration by injection

In one embodiment of the invention the treatment is carried out by administration by injection. Administration by injection includes intravenous, intramuscular, intraarticular, subcutaneous, intradermal, epicutaneous/transdermal and intraperitoneal administration. Compositions for administration via injection may be formulated so as to be suitable for injection by needle or for needleless injection.

Preparation of allergen compositions is generally well known in the art. The allergen may suitably be mixed with excipients which are pharmaceutically acceptable and further compatible with the active ingredient. Examples of suitable excipients are water, saline, dextrose, glycerol, ethanol and the like as well as combinations thereof. The composition may additionally contain other substances such as wetting agents, emulsifying agents, buffering agents or adjuvants enhancing the effectiveness of the composition.

Compositions may suitably be formulated with excipients normally employed for such formulations, e.g. pharmaceutical grades of mannitol, lactose, starch, magnesium stearate, sodium saccharine, cellulose, magnesium carbonate and the like.

The compositions are administered in a way so as to be compatible with the dosage formulation and in such amount as will be therapeutically effective and immunogenic. The quantity of active component contained within the composition depends on the subject to be treated, i.a. the capability of the subjects immune system to respond to the treatment, the route of administration and the age and weight of the subject. In general the treatment may e.g. consist in a treatment protocol, which comprises an up-dosing period, during which the dose is slowly raised, and a maintenance period, wherein the patient receives a fixed maintenance dose. Up-dosing may e.g. comprise 10-20 administrations, carried out weekly or biweekly. In the maintenance dose the patient is treated e.g. monthly or bi-monthly for a period of up to three years. This is contemplated to give desired level of prophylactic or therapeutic effect.

In the case of parenteral administration by e.g. subcutaneous injections, the treatment comprises at least one administration, preferably 1-40 administrations. Suitable dosage ranges can vary within the range from about 0.0001 micro g to 1000 micro g. Expressed as SQ-u the doses may vary from 20 SQ-u to 100000 SQ-u.

In a particular embodiment of the method of the invention, one or more additional rounds of treatment are carried out subsequent to the end of a first treatment round to re-stimulate (boost) the immune system further. Such additional rounds of treatment may e.g. involve a limited number of administrations, e.g. from 1-10, preferably 1-5, over a period of e.g. from one to four weeks. Patients may e.g. be subjected to such additional rounds of treatment one or two times each year. Such a treatment protocol has the advantage of being very effective while at the same time limiting the number of parenteral administrations to a minimum. It is desired to reduce the number of parenteral administrations to a minimum, since such administrations should be performed by specialists and further require post-administration attendance for a period of time.

### Mucosal administration

The mucosa to which the allergy composition is administered may be any suitable mucosa, and the administration includes oral (via the mucosa of the digestive system), nasal, vaginal, sublingual, ocular, rectal, urinal, intramammal, pulmonal, otolar (i.e. via the ear) and buccal administration, preferably buccal or sublingual administration (oromucosal administration). The allergy composition may be in the form of a spray, an aerosol, a mixture, a suspension, a dispersion, an emulsion, a gel, a paste, a syrup, a cream, an ointment, implants (ear, eye, skin, nose, rectal, and vaginal), intramammary preparations, vagitories, suppositories, or uteritories.

It has been speculated that it is preferable to carry out a mucosal administration of a composition via the mucosa, which is subject to the natural exposure to the allergen. Accordingly, for allergies to airborne mucosal antigenic agents, it is preferred to use administration via the respiratory system, preferably an oromucosal administration.

In one embodiment of the invention, the subject is subjected to a vaccination protocol comprising daily administration of the composition. In another embodiment of the invention the vaccination protocol comprises administration of the composition every second day, every third day or every fourth day. For instance, the vaccination protocol comprises administration of the composition for a period of more than 4 weeks, preferably more than 8 weeks, more preferably more than 12 weeks, more preferably more than 16 weeks, more preferably more than 20 weeks, more preferably more than 24 weeks, more preferably more than 30 and most preferably more than 36 weeks.

The period of administration may a continuous period. Alternatively, the period of administration is a discontinuous period interrupted by one or more periods of non-administration. Preferably, the (total) period of non-administration is shorter than the (total) period of administration.

In a further embodiment of the invention, the composition is administered to the patient once a day. Alternatively, the composition is administered to the patient twice a day.

### Oromucosal administration

The immune system is accessible through the oral cavity and sublingual administration of allergens is a known route of administration. Administration may be carried out by placing the composition under the tongue and allowing it to remain there for a short period of time, e.g. from 30 to 300 seconds, preferably from 45 to 240 seconds, more preferably from 60 to 180 seconds, more preferably from 90 to 150 seconds, and most preferably from 90 to 120 seconds.

Oromucosal administration comprises any administration method, wherein the formulation in part or in full comes into contact with the mucosa of the oral cavity and/or the pharynx of the patient. Oromucosal administration methods include sublingual administration and buccal administration.

In one embodiment of the invention, the subject is subjected to a vaccination protocol comprising daily administration of the composition. In another embodiment of the invention the vaccination protocol comprises administration of the composition every second day, every third day or every fourth day. For instance, the vaccination protocol comprises administration of the composition for a period of more than 4 weeks, preferably more than 8 weeks, more preferably more than 12 weeks, more preferably more than 16 weeks, more preferably more than 20 weeks, more preferably more than 24 weeks, more preferably more than 30 and most preferably more than 36 weeks.

The period of administration may a continuous period. Alternatively, the period of administration is a discontinuous period interrupted by one or more periods of non-administration. Preferably, the (total) period of non- administration is shorter than the (total) period of administration.

For treatment of allergies to seasonal allergens, such as grass, weed and tree pollens, the treatment may be given all year round, pre-seasonally, co-seasonally and a combination of pre- and co-seasonally. Pre-seasonal treatment may be given for a period of from 4 to 22 weeks, preferably from 6 to 20 weeks, more preferably from 8 to 18 weeks, more preferably from 9 to 16 weeks, more preferably from 10 to 14 weeks before the start of the season.

In a further embodiment of the invention, the composition is administered to the patient once a day. Alternatively, the composition is administered to the patient twice a day.

The dosage regimen may include an updosing phase, where the dose is increased for each administration, and a maintenance phase, where the same dose is given in each administration. Alternatively, the dosage regimen includes only a maintenance phase, i.e. the subject receives a full dose in the first administration (mono-dose formulation). The number of dosage levels in the updosing phase may be from 2 to 15, preferably from 3 to 12, more preferably from 4-10, more preferably from 5-8.

The oromucosal administration may be carried out using any available oromucosal administration formulation, including a solution, e.g. drops, a suspension, a solid dosage forms, e.g. a fast dispersing solid dosage form, and lozenges.

In a particular embodiment of the invention, sublingual immunotherapy (SLIT) is used, in which case fast dispersing dosage forms, drops and lozenges are preferred formulations. In a particular embodiment, the oromucosal formulation is selected from the group consisting of a solution and a solid dosage form.

Examples of fast dispersing dosage forms are those disclosed in US-A- 5,648,093, WO 00/51568, WO 02/13858, WO99/21579, WO 00/44351, US-A- 4,371,516 and EP-278 877, as well as WO 2004/047794 and WO 2004/075875 filed in the assignee name of ALK-Abelló A/S. Preferred fast dispersing dosage forms are those produced by freeze-drying. Preferred matrix forming agents are fish gelatine and modified starch.

Allergy compositions in the form of an aqueous solution or a fast-dispersing tablet, cf. WO 04/047794, are particularly suitable for buccal and sublingual administration.

The preferred potency of a mono-dose formulation intended for daily administration is from 1 to 2000 STU, more preferable from 50 to 1000 STU, more preferably from 100 to 500 STU, more preferable from 130 to 350 STU and most preferable from 150 to 250 STU.

The preferred potency of a mono-dose formulation intended for daily administration is from 100 to 500000 SQ-u, more preferably from 200 to 300000 SQ-u, more preferable from 500 to 200000 SQ-u, more preferably from 1000 to 100000 SQ-u, more preferable from 1500 to 80000 SQ-u and most preferable from 2000 to 50000 SQ-u.

The amount of allergen, which corresponds to a given level of potency, varies strongly depending on the allergen specie. In a further embodiment of the invention the concentration of major allergen in a mono-dose intended for daily administration is from 0.05 to 50 micro g, more preferably from 0.05 micro g to 30 micro g, more preferably from 0.06 micro g to 25 micro g, more preferably from 0.07 micro g to 20 micro g, more preferably from 0.08 micro g to 15 micro g, more preferably from 0.09 micro g to 10 micro g and most preferably from 0.1 micro g to 7 micro g.

Incremental dosage desensitisation, where the dose of allergen in the form of a fast dispersing solid dosage form, is increased to a certain maximum, may be used in the present invention. The preferred potency of a unit dose of the dosage form is from 150 - 1000000 SQ-u/dosage form, more preferred the potency is from 500 - 500000 SQ-u/dosage form and more preferably the potency is from 1000 - 250000 SQ-u/dosage form, even more preferred 1500- 125000 SQ-u/dosage form most preferable 1500-75000 SQ-u/dosage form.

In another embodiment of the invention the dosage form is a repeated mono- dose, preferably within the range of 1500-75000 SQ-u/dosage form.

In a particular embodiment of the method of the invention the first allergen is not administered to the respiratory tract, e.g. by intranasal administration, within a period coinciding partly or entirely with the subject's exposure to the second biological source material. Also, in a particular embodiment of the method disclosed herein the first and second allergens are not co-administered to the subject.

### Composition formulation

It is to be understood that the composition for the use of the invention may further comprise additional adjuvants and other excipients suitable for the selected type of formulation. Such additional adjuvants and excipients are well-known to the person skilled in the art and include i.a. solvents, emulsifiers, wetting agents, plasticizers, colouring substances, fillers, preservatives, viscosity adjusting agents, buffering agents, mucoadhesive substances, and the like. Examples of formulation strategies are well-known to the person skilled in the art.

The composition for the use of the invention comprises at least one first allergen. A specific embodiment of the composition of the invention comprises one or two first allergens. Another specific embodiment of the composition of the invention comprises one first allergen.

### Adjuvant

The allergy composition may include an adjuvant, which may be any conventional adjuvant, including oxygen-containing metal salts, heat-labile enterotoxin (LT), cholera toxin (CT), cholera toxin B subunit (CTB), polymerised liposomes, mutant toxins, e.g. LTK63 and LTR72, microcapsules, interleukins (e.g. IL-lbeta, IL-2, IL-7, IL-12, INFGAMMA), GM-CSF, MDF derivatives, CpG oligonucleotides, LPS, MPL, phosphophazenes, Adju-Phos(R), glucan, antigen formulation, liposomes, DDE, DHEA, DMPC, DMPG, DOC/Alum Complex, Freund's incomplete adjuvant, ISCOMs(R), LT Oral Adjuvant, muramyl dipeptide, monophosphoryl lipid A, muramyl tripeptide, and phospatidylethanolamine.

The oxygen-containing metal salt may be any oxygen-containing metal salt providing the desired effect. In a preferred embodiment, the cation of the oxygen-containing metal salt is selected from Al, K, Ca, Mg, Zn, Ba, Na, Li, B, Be, Fe, Si, Co, Cu, Ni, Ag, Au, and Cr. In a preferred embodiment, the anion of the oxygen-containing metal salt is selected from sulphates, hydroxides, phosphates, nitrates, iodates, bromates, carbonates, hydrates, acetates, citrates, oxalates, and tartrates, and mixed forms thereof. Examples are aluminium hydroxide, aluminium phosphate, aluminium sulphate, potassium aluminium sulphate, calcium phosphate, Maalox (mixture of aluminium hydroxide and magnesium hydroxide), beryllium hydroxide, zinc hydroxide, zinc carbonate, zinc chloride, and barium sulphate.

### Prophylactic treatment of asthma

Herein is further disclosed a composition comprising a first allergen from a first biological source material group for prophylactic treatment of asthma, wherein the subject has not developed any clinical symptoms of allergy to the first allergen, and wherein the subject has a negative skin prick test to all of the food compositions hens' egg white, cows' milk, peanut and soy bean.

The subject may have a negative skin prick test to the first allergen.

The subject might not exhibit a positive IgE response to the first allergen.

The subject might not exhibit a positive IgE response to any of the food compositions hens' egg white, cows' milk, peanut and soy bean.

Also disclosed is a method of prophylactic treatment of asthma comprising administering to the subject a composition comprising a first allergen from a first biological source material group, wherein the subject has not developed any clinical symptoms of allergy to the first allergen, and wherein the subject has a negative skin prick test to all of the food compositions hens' egg white, cows' milk, peanut and soy bean.

### DEFINITIONS

The expression "prophylactic treatment of allergy to at least one second allergen" means a treatment with a composition comprising at least one first allergen, which prevents or postpones sensitisation of the subject to at least one second allergen, or which prevents, ameliorates or postpones clinical symptoms of allergy to at least one second allergen, or which prevents, alleviates or postpones clinical symptoms of asthma.

The expression "prophylactic treatment of asthma" means a treatment with a composition comprising at least one first allergen, which prevents, alleviates or postpones clinical symptoms of asthma.

The expression "clinical symptoms of allergy" means any conventional symptom of allergy to an allergen.

The expression "clinical symptoms of asthma" means any conventional symptom of asthma.

The expression "sensitisation" means that the subject has a positive IgE response and/or a positive skin prick test to the second allergen in question.

The expression "IgE response" means an IgE response specific to the allergen in question.

The expression "positive IgE response" means a positive response in the specific IgE assay ImmunoCAP® marketed by Phadia, which means a measurement above the detection level of the assay (0.35 kUA/l).

The expression "positive Skin Prick Test" means a mean wheal diameter equal to or larger than 3 mm when compared to a control of a physiological saline solution, wherein the Skin Prick Test used is Soluprick® SQ marketed by ALK-Abelló A/S.

The expression "respiratoy allergen" means any allergen, which is airborne and which may come into contact with the mucosa of a subject by respiration.

The expression "venomous allergen" means any allergen, which is present in a venom, e.g. a venom from a bee, a wasp and an ant.

The expression "food composition" means any food containing one or more allergens capable of causing allergy, including IgE mediated (Type I) and non-IgE mediated (Type IV) mediated reactions.

The expression "food allergen" is an allergen present in a food composition.

The expression "taxonomic order system" means the Linnaean taxonomic system as listed on http://www.ncbi.nlm.nih.gov/Taxonomy/Browser/wwwtax.cgi.

The expression "allergen" means any allergen composition capable of causing allergy, including IgE mediated (Type I) and non-IgE mediated (Type IV) mediated reactions, or asthma.

The expression "first allergen" means any allergen used as active substance in the prophylactic treatment disclosed herein.

The expression "second allergen" means any allergen capable of causing clinical symptoms, which are treated in the prophylactic treatment disclosed herein.

The expression "the subject has not developed any clinical symptoms of allergy" means that the subject has not exhibited clinical symptoms of allergy at any time before the start of treatment.

### EXAMPLES

### Example 1: Randomised controlled trial of primary prevention of atopy using house dust mite allergen oral immunotherapy in early childhood

### Subjects treated

A randomised, double-blind, placebo-controlled study was conducted. 111 infants aged 5 to 9 months were enrolled. Inclusion criteria were infants at high risk of developing atopy based on heredity, as defined by two or more first degree relatives, i.e. biological mother, father or sibling, affected by asthma or allergic rhinoconjunctivitis or ezcema. Exclusion criteria for the infants enrolled were infants wit a positive skin prick test to one or more of six common allergens: house dust mite, grass pollen, cat, cows' milk, egg white and peanut. Premature infants (<37 weeks of gestation) and those with major concurrent health problems (e.g. congenital heart disease, cystic fibrosis, those requiring special feeding regimens) were also excluded.

### Allergen composition and dose

The infants treated were given either placebo or active prophylactic treatment. The medicament used in active prophylactic treatment was a glycerinated allergen extract of house dust mite developed for allergen-specific immunotherapy treatment of HDM allergy (SLITone^{PLUS®} HDM 2500 STU/ml). The medicament is derived from extraction and purification of the source material: purified mite body (with culture fractions) of the species *Dermatophagoides pteronyssius* and *Dermatophagoides farina* in equal parts (1.1 mixture). The medicament is formulated as drops for oromucosal administration. The concentration in one vial is 500 STU (200 µl) (standard treatment units). For the present trial, 4 vials are administered every day, 2 in the morning and 2 in the evening, corresponding to 2000 STU per day. No up-dosing was carried out. The composition of the medicament is set out in Table 1.

**Table 1:**

| **Ingredient** | **Function** | **Reference** |
|---|---|---|
| Allergen extract | Active ingredient | In-House reference |
| Glycerol | Stabilizer / viscosity | Ph. Eur. |
| Sodium Chloride | Ionic Strength | Ph. Eur. |
| Sisodium phosphate dihydrate | Buffer | Ph. Eur. |
| Sodium dihydrogen phosphate, dihydrate | Buffer | Ph. Eur. |
| Sodium hydroxide and/or hydrochloric acid | pH adjustment | Ph. Eur. |
| Water for injection | Solvent | Ph. Eur. |

### Methods

### Skin Prick Test

A commercial test (Soluprick® SQ marketed by ALK-Abelló A/S) was used for the testing. In short, droplets of liquid allergen test preparations and controls are applied to the skin the forearm or the back. The skin should be dry and clean and preferably disinfected with alcohol. Then, the top skin layer is pierced with a lancet through the droplet. The result is read after 15 minutes of reaction. The test was considered to be positive, if the mean weal diameter is equal to or higher than 3 mm when compared to a saline control. The "mean weal diameter" is determined by measuring the longest diameter of the weal and the diameter perpendicular to that and calculating the mean value of the said two measurements. The expression "compared to a saline control" means that the mean weal diameter resulting from the negative saline control is determined and subtracted from the mean weal diameter of allergen test preparation. Usually, the mean weal diameter resulting from the saline liquid is zero.

### IgE and IgG measurements

### Treatment timelines, groups, samples and tests

The treatment group included 53 infants and the placebo group included 51 infants. Infants in the treatment group received a total daily dose of 2000 STU every day for 12 months. An assessment of the infants was carried out at the start of treatment (baseline assessment), and subsequently was perfomed at month 3, 6, 9 and 12 months after the start of treatment. At each assessment a Skin Prick Test was carried out for each of the six allergens house dust mite, grass pollen, cat, cows' milk, egg white and peanut. Also, clinical symptoms were assessed by validated questionnaires (e.g. ASAAC, SCORAD) and /or clinical assessment of wheeze, recurrent wheeze, eczema, food allergy and anaphylaxis.

### Results

### Skin Prick Test (SPT)

The results are listed in Table 2, which indicates the number of infants, which has a positive Skin Prick Test at the end of the treatment period with respect to each of the six allergens house dust mite, grass pollen, cat, cows' milk, egg white and peanut as well as a cumulative value for the group of the said six allergens.

**Table 2: Level of infants with a positive Skin Prick Test (SPT)**

| **Allergen** | **Treatment Group (n=53)** | **Placebo Group (n=51)** | **p-value (Fichers exact test)** |
|---|---|---|---|
| House Dust Mite | 3 (5.7 %) | 4 (7.8 %) | NA |
| Grass Pollen | 2 (3.7 %) | 1 (2.0 %) | NA |
| Cat | 1 (1.9 %) | 2 (3.9 %) | NA |
| Cows' Milk | 1 (1.9 %) | 2 (3.9 %) | NA |
| Egg White | 1 (1.9 %) | 2 (3.9 %) | NA |
| Peanut | 1 (1.9 %) | 2 (3.9 %) | NA |
| Positive SPT to one or more of six allergens (Cumulative value) | 5 (9.4 %) | 13 (25.5 %) | 0.039 |

As will appear from the results of Table 2 for the six individual allergens, there is a clear general trend that the level of infants with a positive SPT is lower for the treatment group than for the placebo group. Due to the low number of infants with a positive SPT for the six individual allergens, the trend cannot be verified as statistically significant. However, the cumulative value for all six allergens shows with statistical sigfificance that the level of infants with a positive SPT is lower for the treatment group than for the placebo group.

In conclusion, the present clinical study shows that prophylactic treatment with House Dust Mite allergen reduces the proportion of sensitisation in a group of subjects, both to the House Dust Mite allergen as well as to five other allergens and food compostions. The prevention of sensitisation in a number of subjects in the treatment group means that for those subjects the sensitisation is delayed or prevented altogether, which again means that a subsequent development of clinical symptoms of allergy is likewise delayed or prevented altogether.

### Eczema

Eight infants (15 %) in the treatment group and three infants (6 %) in the placebo group had eczema recorded at the baseline prior to the start of treatment. At the end of the study there were 17 participants in each group with eczema symptoms recorded, p-value = 0.231.

### Wheeze

Eighteen infants (34 %) in the treatment group and thirteen infants (25 %) in the placebo group had reported wheeze at the baseline prior to the start of treatment. At the end of the study there were 36 participants (68 %) and 35 participants (69 %) in the treatment and placebo group, respectively, with wheeze symptoms recorded, p-value = 0.5631.

## Claims

1. A composition comprising at least one first allergen, for use in prophylactic treatment of allergy to at least one second allergen in a human subject,
wherein the first allergen is a respiratory allergen of a first biological source material group of the genus Dermatophagoides,
wherein the second allergen is selected from the group consisting of a respiratory allergen of a second biological source material group, a venomous allergen of a second biological source material group, and a food allergen of a food composition,
wherein the human subject
i. has a negative skin prick test to the first allergen and to the second allergen and
ii. has not developed any clinical symptoms of allergy to the first allergen and to the second allergen, and
wherein the first and second biological source material groups are not from the same order of the taxonomic order system.

2. The composition for use according to claim 1, wherein the first and second allergen are unrelated in function, amino acid sequence and structure.

3. The composition for use according to claim 1 or 2, wherein the human subject does not exhibit a positive IgE response to the first allergen and/or to the second allergen.

4. The composition for use according to any one of claims 1-3, wherein the second biological source material group for a respiratory allergergen is a genus selected from the group consisting of the genera from the grass family Poaceae, the daisy family Asteraceae, the birch family Betulaceae, the beech family Fagaceae, the cypress family Cupressaceae, and the cat family Felidae.

5. The composition for use according to any one of claims 1-3, wherein the second biological source material group for a venom allergen is a genus selected from the group consisting of the genera from the bee family Apidae and the wasp family Vespidae.

6. The composition for use according to any one of claims 1-3, wherein the second biological source material group for the respiratory allergen is a genus selected from the group consisting of the genera Phleum, Lolium, Poa, Cynodon, Dactylis, Holcus, Sorghum, Phalaris, Secale, Ambrosia, Betula, Alnus, Corylius, Carpinus, Quercus, Juniperus, and Cryptomeria.

7. The composition for use according to any one of claims 1-3, wherein the second biological source material group for a respiratory allergen is a genus selected from the group consisting of the genera Phleum, Lolium, Poa, Cynodon, Dactylis, Holcus, Sorghum, Phalaris, and Secale.

8. The composition for use according to any one of claims 1-3, wherein the food composition component of the second biological source material group is selected from the group consisting of hens' egg, cows' milk, and peanut.

9. The composition for use according to any one of claims 1-3, wherein the second biological source material group is of the genus Felis.

10. The composition for use according to any one of claims 1-3, wherein the second biological source material group for a venomous allergen is of the genus Apis or of the genus Vespula.

11. The composition for use according to any one of claims 1-3, wherein the second biological source material is selected from the group consisting of the genera of the taxonomic order system Phleum and Felis and from the food compositions hens' egg, cows' milk, and peanut.

12. The composition for use according to any one of the preceding claims, wherein the subject has not developed any clinical symptoms of allergy to any of house dust mite, grass pollen, cat, hens' eggs, cow's milk, and peanut.

13. The composition for use according to any one of the preceding claims, wherein the first allergen of the composition is an allergen extract of the first biological source material..

14. The composition for use according to any one of the preceding claims, wherein the composition is administered by oromucosal administration.

15. The composition for use according to any one of the preceding claims, wherein the prophylactic treatment comprises preventing or delaying sensitisation to the second allergen.

## Patentansprüche

1. Zusammensetzung, die mindestens ein erstes Allergen umfasst, zur Anwendung in der vorbeugenden Behandlung von Allergie gegen mindestens ein zweites Allergen bei einem menschlichen Subjekt,
wobei das erste Allergen ein Atemwegsallergen aus einer ersten Gruppe von biologischem Quellmaterial aus der Gattung Dermatophagoides ist,
wobei das zweite Allergen ausgewählt ist aus der Gruppe bestehend aus einem Atemwegsallergen aus einer zweiten Gruppe von biologischem Quellmaterial, einem Giftallergen aus einer zweiten Gruppe von biologischem Quellmaterial und einem Lebensmittelallergen aus einer Nahrungsmittelzusammensetzung,
wobei das menschliche Subjekt
i. einen negativen Prick-Hauttest auf das erste Allergen und auf das zweite Allergen aufweist und
ii. keine klinischen Symptome einer Allergie auf das erste Allergen und auf das zweite Allergen entwickelt hat und
wobei die erste und die zweite Gruppe von biologischem Quellmaterial nicht von derselben Ordnung des taxonomischen Ordnungssystems sind.

2. Zusammensetzung zur Anwendung gemäß Anspruch 1, wobei das erste und das zweite Allergen in ihrer Funktion, Aminosäuresequenz und Struktur nicht verwandt sind.

3. Zusammensetzung zur Anwendung gemäß Anspruch 1 oder 2, wobei das menschliche Subjekt keine positive IgE-Antwort auf das erste Allergen und/oder auf das zweite Allergen zeigt.

4. Zusammensetzung zur Anwendung gemäß einem der Ansprüche 1-3, wobei die zweite Gruppe von biologischem Quellmaterial für ein Atemwegsallergen eine Gattung ist, die ausgewählt ist aus der Gruppe bestehend aus den Gattungen der Grasfamilie Poaceae, der Korbblütlerfamilie Asteraceae, der Birkenfamilie Betulaceae, der Buchenfamilie Fagaceae, der Zypressenfamilie Cupressaceae und der Katzenfamilie Felidae.

5. Zusammensetzung zur Anwendung gemäß einem der Ansprüche 1-3, wobei die zweite Gruppe von biologischem Quellmaterial für ein Giftallergen eine Gattung ist, die ausgewählt ist aus der Gruppe bestehend aus den Gattungen der Bienenfamilie Apidae und der Wespenfamilie Vespidae.

6. Zusammensetzung zur Anwendung gemäß einem der Ansprüche 1-3, wobei die zweite Gruppe von biologischem Quellmaterial für das Atemwegsallergen eine Gattung ist, die ausgewählt ist aus der Gruppe bestehend aus den Gattungen Phleum, Lolium, Poa, Cynodon, Dactylis, Holcus, Sorghum, Phalaris, Secale, Ambrosia, Betula, Alnus, Corylius, Carpinus, Quercus, Juniperus und Cryptomeria.

7. Zusammensetzung zur Anwendung gemäß einem der Ansprüche 1-3, wobei die zweite Gruppe von biologischem Quellmaterial für ein Atemwegsallergen eine Gattung ist, die ausgewählt ist aus der Gruppe bestehend aus den Gattungen Phleum, Lolium, Poa, Cynodon, Dactylis, Holcus, Sorghum, Phalaris und Secale.

8. Zusammensetzung zur Anwendung gemäß einem der Ansprüche 1-3, wobei die Komponente der Nahrungsmittelzusammensetzung der zweiten Gruppe von biologischem Quellmaterial ausgewählt ist aus der Gruppe bestehend aus Hühnerei, Kuhmilch und Erdnuss.

9. Zusammensetzung zur Anwendung gemäß einem der Ansprüche 1-3, wobei die zweite Gruppe von biologischem Quellmaterial von der Gattung Felis ist.

10. Zusammensetzung zur Anwendung gemäß einem der Ansprüche 1-3, wobei die zweite Gruppe von biologischem Quellmaterial für ein Giftallergen von der Gattung Apis oder von der Gattung Vespula ist.

11. Zusammensetzung zur Anwendung gemäß einem der Ansprüche 1-3, wobei das zweite biologische Quellmaterial ausgewählt ist aus der Gruppe bestehend aus den Gattungen des taxonomischen Ordnungssystems Phleum und Felis und aus den Nahrungsmittelzusammensetzungen mit Hühnerei, Kuhmilch und Erdnuss.

12. Zusammensetzung zur Anwendung gemäß einem der vorangehenden Ansprüche, wobei das Subjekt keine klinischen Symptome einer Allergie gegen eines von Hausstaubmilbe, Graspollen, Katze, Hühnerei, Kuhmilch und Erdnuss entwickelt hat.

13. Zusammensetzung zur Anwendung gemäß einem der vorangehenden Ansprüche, wobei das erste Allergen der Zusammensetzung ein Allergenextrakt von dem ersten biologischen Quellmaterial ist.

14. Zusammensetzung zur Anwendung gemäß einem der vorangehenden Ansprüche, wobei die Zusammensetzung mittels oromukosaler Verabreichung verabreicht wird.

15. Zusammensetzung zur Anwendung gemäß einem der vorangehenden Ansprüche, wobei die vorbeugende Behandlung das Vermeiden oder Aufschieben einer Sensibilisierung gegen das Allergen umfasst.

## Revendications

1. Composition comprenant au moins un premier allergène, pour son utilisation dans le traitement prophylactique de l'allergie à au moins un deuxième allergène chez un sujet humain,
dans laquelle le premier allergène est un allergène respiratoire d'un premier groupe de substances sources biologiques du genre Dermatophagoides,
dans laquelle le deuxième allergène est sélectionné dans le groupe consistant en un allergène respiratoire d'un deuxième groupe de substances sources biologiques, un allergène venimeux d'un deuxième groupe de substances sources biologiques, et un allergène alimentaire d'une composition alimentaire,
dans laquelle le sujet humain
i. a un prick-test cutané négatif au premier allergène et au deuxième allergène et
ii. n'a pas développé de symptômes cliniques d'allergie au premier allergène et au deuxième allergène, et
dans lequel les premier et deuxième groupes de substances sources biologiques ne sont pas du même ordre du système d'ordres taxonomiques.

2. Composition pour son utilisation selon la revendication 1, dans laquelle le premier et le deuxième allergène n'ont pas de lien relativement à la fonction, la séquence d'acides aminés et la structure.

3. Composition pour son utilisation selon la revendication 1 ou 2, dans laquelle le sujet humain ne présente pas de réponse des IgE positive au premier allergène et/ou au deuxième allergène.

4. Composition pour son utilisation selon l'une quelconque des revendications 1 à 3, dans laquelle le deuxième groupe de substances sources biologiques pour un allergène respiratoire est un genre sélectionné dans le groupe consistant en les genres de la famille des herbacées Poaceae, de la famille des astéracées Asteraceae, de la famille des bétulacées Betulaceae, de la famille des fagacées Fagaceae, de la famille des cupressacées Cupressaceae, et de la famille des félidés Felidae.

5. Composition pour son utilisation selon l'une quelconque des revendications 1 à 3, dans laquelle le deuxième groupe de substances sources biologiques pour un allergène venimeux est un genre sélectionné dans le groupe consistant en les genres de la famille des abeilles Apidae et de la famille des guêpes Vespidae.

6. Composition pour son utilisation selon l'une quelconque des revendications 1 à 3, dans laquelle le deuxième groupe de substances sources biologiques pour l'allergène respiratoire est un genre sélectionné dans le groupe consistant en les genres Phleum, Lolium, Poa, Cynodon, Dactylis, Holcus, Sorghum, Phalaris, Secale, Ambrosia, Betula, Alnus, Corylius, Carpinus, Quercus, Juniperus, et Cryptomeria.

7. Composition pour son utilisation selon l'une quelconque des revendications 1 à 3, dans laquelle le deuxième groupe de substances sources biologiques pour un allergène respiratoire est un genre sélectionné dans le groupe consistant en les genres Phleum, Lolium, Poa, Cynodon, Dactylis, Holcus, Sorghum, Phalaris et Secale.

8. Composition pour son utilisation selon l'une quelconque des revendications 1 à 3, dans laquelle le composant de composition alimentaire du deuxième groupe de substances sources biologiques est sélectionné dans le groupe consistant en les œufs de poule, le lait de vache et les cacahuètes.

9. Composition pour son utilisation selon l'une quelconque des revendications 1 à 3, dans laquelle le deuxième groupe de substances sources biologiques est du genre Felis.

10. Composition pour son utilisation selon l'une quelconque des revendications 1 à 3, dans laquelle le deuxième groupe de substances sources biologiques pour un allergène venimeux est du genre Apis ou du genre Vespula.

11. Composition pour son utilisation selon l'une quelconque des revendications 1 à 3, dans laquelle la deuxième substance source biologique est sélectionnée parmi le groupe consistant en les genres du système d'ordres taxonomiques Phleum et Felis et parmi les compositions alimentaires œufs de poule, lait de vache et cacahuètes.

12. Composition pour son utilisation selon l'une quelconque des revendications précédentes, dans laquelle le sujet n'a pas développé de signes cliniques d'allergie à l'un quelconque des acariens de la poussière domestique, du pollen de l'herbe, des chats, des œufs de poule, du lait de vache, et des cacahuètes.

13. Composition pour son utilisation selon l'une quelconque des revendications précédentes, dans laquelle le premier allergène de la composition est un extrait d'allergène de la première substance source biologique.

14. Composition pour son utilisation selon l'une quelconque des revendications précédentes, dans laquelle la composition est administrée par administration oromuqueuse.

15. Composition pour son utilisation selon l'une quelconque des revendications précédentes, dans laquelle le traitement prophylactique comprend la prévention ou le ralentissement de la sensibilisation au second allergène.
